# EUROPÄISCHE PATENTSCHRIFT

(11) **EP 4 162 263 B1**
(45) Veröffentlichungstag und Bekanntmachung des Hinweises auf die Patenterteilung: **25.03.2026**
(21) Anmeldenummer: 21730146.4
(22) Anmeldetag: 27.05.2021
(51) Int. Cl.: G01N 27/16, G01N 33/00

(54) **VERWENDUNG EINES GASMESSGERÄT UND VERFAHREN ZUM MESSEN VON DICYAN IN ANWESENHEIT VON CYANWASSERSTOFF**
USE OF A GAS MEASURING DEVICE AND METHOD FOR MEASURING DICYANO IN THE PRESENCE OF HYDROGEN CYANIDE
UTILISATION D'UN APPAREIL DE MESURE DE GAZ ET PROCÉDÉ DE MESURE DE DICYAN EN PRÉSENCE DE CYANURE D'HYDROGÈNE

(30) Priorität: 05.06.2020 DE 102020114982
(43) Veröffentlichungstag der Anmeldung: 12.04.2023
(73) Patentinhaber: Dräger Safety AG & Co. KGaA, 23560 Lübeck (DE)
(72) Erfinder: HAUPT, Stephan, 23558 Lübeck (DE); NAUBER, Andreas, 23558 Lübeck (DE); SICK, Michael, 23558 Lübeck (DE); REIER, Tobias, 23558 Lübeck (DE); RITTEMANN, Steffen, 23558 Lübeck (DE)
(86) Internationale Anmeldenummer: PCT/EP2021/064203
(87) Internationale Veröffentlichungsnummer: WO 2021/244941

(56) Entgegenhaltungen:
- US-A- 4 703 646
- US-A- 6 001 383
- US-A1- 2002 043 458
- US-A1- 2018 328 873

## Beschreibung

Die vorliegende Erfindung betrifft eine Verwendung eines Gasmessgeräts zum Messen von Dicyan in Anwesenheit von Cyanwasserstoff und ein Verfahren zum Messen von Dicyan in Anwesenheit von Cyanwasserstoff.

### STAND DER TECHNIK

Die Fumigation, also die Desinfektion durch Ausräuchern, ist in der Landwirtschaft eine gängige Methode zum Abtöten von Keimen und Bakterien auf Produkten, die später in der Lebensmittelindustrie verwendet und verkauft werden.

In der Vergangenheit wurde die Fumigation häufig mit Methylbromid durchgeführt. Aufgrund der stark krebserregenden Wirkung ist die Anwendung von Methylbromid zur Fumigation bereits in einigen Ländern verboten. Als Alternative hat sich Dicyan als wirksam erwiesen.

Da Dicyan bei einer Begasung häufig zusammen mit Cyanwasserstoff (HCN) auftritt, besteht Bedarf zur Messung von Dicyan in Anwesenheit von Cyanwasserstoff.

Zum Messen von Dicyan werden in der Regel Halbleitersensoren verwendet, die jedoch eine eingeschränkte Sensitivität und Stabilität zeigen und, dadurch bedingt, nicht oder nur bedingt zur Detektion einer Überschreitung eines Arbeitsplatzgrenzwerts von 5 ppm geeignet sind.

Weiterhin kann Dicyan durch ein Massenspektrometer gemessen werden. Ein solches Verfahren wird in der US6001383 A offenbart. Da Massenspektrometer jedoch unhandlich und kompliziert in der Anwendung sind, sind diese ebenfalls nicht oder nur bedingt zur Detektion einer Überschreitung eines Arbeitsplatzgrenzwerts von 5 ppm geeignet.

Die JP 2008 076 235 A beschreibt ein Verfahren zum Messen von Dicyan bei dem Hydrogensulfide aus einer Probe vaporisiert werden, um schließlich die Probe mittels eines Hydrogen-cyanid-Gassensors zu messen.

US 2018328873 A1 offenbart einen Sensor zum Detektieren von Stickstofftrifluorid, welches katalytisch zu Stickoxiden gespalten wird.

### OFFENBARUNG DER ERFINDUNG

Ausgehend von dem voranstehend beschriebenen Stand der Technik hat der Erfindung die Aufgabe zugrunde gelegen, eine Möglichkeit zum Messen von Dicyan bereitzustellen, die diese Nachteile zumindest teilweise nicht aufweist. Es ist daher eine Aufgabe der vorliegenden Erfindung, eine Möglichkeit zum verlässlichen und einfachen Erkennen eines Überschreitens eines erlaubten Arbeitsplatzgrenzwerts von Dicyan bereitzustellen.

Voranstehende Aufgabe wird gelöst durch den Gegenstand der jeweiligen unabhängigen Ansprüche. Weitere Merkmale und Details der Erfindung ergeben sich aus den Unteransprüchen, der Beschreibung und den Zeichnungen. Dabei gelten Merkmale und Details, die im Zusammenhang mit dem Gasmessgerät beschrieben sind, selbstverständlich auch im Zusammenhang mit dem erfindungsgemäßen Verfahren und jeweils umgekehrt, sodass bezüglich der Offenbarung zu den einzelnen Erfindungsaspekten stets wechselseitig Bezug genommen wird beziehungsweise werden kann.

Es wird gemäß einem ersten Aspekt zur Lösung der Aufgabe die Verwendung eines Gasmessgeräts zum Messen von Dicyan in Anwesenheit von Cyanwasserstoff vorgestellt. Das Gasmessgerät umfasst eine Messkammer, ein Heizelement und einen elektrochemischen Sensor. Die Messkammer umfasst eine Probe aufweisend Dicyan. Das Heizelement ist dazu konfiguriert, in der Probe enthaltenes Dicyan thermisch in Spaltprodukte zu spalten. Der Sensor ist dazu konfiguriert, die durch das thermische Spalten erhaltenen Spaltprodukte des Dicyans zu detektieren.

Das erfindungsgemäß eingesetzte Gasmessgerät ist dazu konfiguriert, eine Konzentration von Dicyan in der Probe auch bei Anwesenheit von Cyanwasserstoff verlässlich bzw. selektiv und mit einer hohen Sensitivität, insbesondere einer Sensitivität, die es erlaubt Dicyan ab einer Konzentration von 1 ppm sicher nachzuweisen, zu bestimmen.

Zum Messen einer Konzentration Dicyan in der Probe umfasst das Gasmessgerät einen elektrochemischen Hauptsensor, der in Kombination mit einem Heizelement, wie bspw. einem Heizdraht oder einer Heizplatte eingesetzt wird.

Da ein elektrochemischer Sensor Dicyan nicht direkt detektieren bzw. messen kann, ist erfindungsgemäß vorgesehen, dass die jeweiligen in der Probe befindlichen Dicyanmoleküle mittels des Heizelements thermisch gespalten werden. Insbesondere führt das Heizelement eine Pyrolyse der Dicyanmoleküle durch. Durch das thermische Spalten entstehen Spaltprodukte, wie bspw. Stickoxide oder Cyanwasserstoff, die von dem erfindungsgemäß vorgesehenen elektrochemischen Hauptsensor erfasst werden können.

Es kann vorgesehen sein, dass eine Temperatur, die mittels des Heizelements zum thermischen Spalten jeweiliger Dicyanmoleküle eingestellt wird, in Abhängigkeit jeweiliger in der Kammer des vorgestellten Gasmessgeräts vorliegender Reaktionsbedingungen gewählt wird. Insbesondere kann die Temperatur in Abhängigkeit jeweiliger in der Kammer vorhandener Katalysatoren eingestellt werden. Alternativ oder zusätzlich ist denkbar, dass die Temperatur in Abhängigkeit weiterer Reaktionsbedingungen, wie bspw. einer in der Kammer vorliegenden relativen Luftfeuchtigkeit, eingestellt wird.

Das erfindungsgemäß vorgesehene Heizelement kann frei in der Kammer des vorgestellten Gasmessgeräts angeordnet sein. Dies bedeutet, dass das Heizelement und der Sensor getrennt voneinander angeordnet sein können. Alternativ ist auch eine kombinierte bzw. integrierte Anordnung von Heizelement und Sensor in einem einzelnen bzw. kombinierten Bauteil möglich. Es kann weiterhin vorgesehen sein, dass das Heizelement dazu konfiguriert ist, in der Probe enthaltenes Dicyan zumindest teilweise in Stickoxide zu spalten, und dass der Sensor dazu konfiguriert ist, Stickoxide zu detektieren.

Da Stickoxide, wie bspw. Sickstoffmonoxid oder Stickstoffdioxid, einfach und genau mittels eines elektrochemischen Sensors detektierbar sind, eignet sich eine Einstellung des Heizelements auf einen Temperaturbereich zum Spalten von Dicyan in Stickoxide, d. h. in Sickstoffmonoxid und/oder Stickstoffdioxid, besonders vorteilhaft zum Betrieb des Gasmessgeräts.

Es kann weiterhin vorgesehen sein, dass das Gasmessgerät eine Recheneinheit umfasst, die dazu konfiguriert ist, anhand von durch den Sensor ermittelten Messwerten eine Konzentration von in der Probe enthaltenem Cyanwasserstoff zu berechnen. Alternativ kann vorgesehen sein, dass das Gasmessgerät eine Recheneinheit umfasst und der Sensor dazu konfiguriert ist, Cyanwasserstoff zu detektieren, wobei die Recheneinheit dazu konfiguriert ist, anhand von während eines ersten Zeitbereichs vor einer thermischen Spaltung durch das Heizelement mittels des Sensors ermittelten Messwerten eine Konzentration von in der Probe enthaltenem Cyanwasserstoff und anhand von während eines zweiten Zeitbereichs nach einer thermischen Spaltung durch das Heizelement mittels des Sensors ermittelten Messwerten eine Konzentration von in der Probe enthaltenem Dicyan zu berechnen.

Mittels einer Recheneinheit, wie bspw. einem Computer oder jeder anderen Form von programmierbarer Schaltung, kann unter Verwendung bspw. eines vorgegebenen Koeffizienten von durch den Sensor des Gasmessgeräts ermittelten Messwerten auf eine Konzentration von Dicyan in einer jeweiligen Probe geschlossen werden. Um den Koeffizienten zu ermitteln oder zu aktualisieren, kann der Sensor bspw. anhand einer Kalibrierungsprobe kalibriert werden.

Der Sensor kann sensitiv für Cyanwasserstoff sein, wobei dann, um eine Wechselwirkung von in einer jeweiligen Probe bereits vorhandenem Cyanwasserstoff mit durch einen thermischen Spaltvorgang erzeugtem Cyanwasserstoff zu vermeiden, vorgesehen ist, dass die in der Probe bereits vorhandene Konzentration an Cyanwasserstoff vor dem thermischen Spaltvorgang bestimmt wird. Entsprechend kann anhand eines Unterschieds zwischen Messwerten, die vor dem thermischen Spaltvorgang und nach dem thermischen Spaltvorgang ermittelt wurden, auf eine Konzentration an Dicyan, das in Cyanwasserstoff gespalten wurde, geschlossen und auf eine in der Probe ursprünglich vorhandene Konzentration an Cyanwasserstoff geschlossen werden.

Das Gasmessgerät weist eine Oberfläche auf, die als Katalysator bei einer thermischen Spaltung von Dicyan wirkt.

Mittels eines Katalysators bzw. einer katalytischen Oberfläche kann eine Temperatur, die von dem erfindungsgemäß vorgesehenen Heizelement für einen thermischen Spaltvorgang von Dicyanmolekülen bereitzustellen ist, reduziert werden. Weiterhin können durch eine geeignete Auswahl eines Materials einer entsprechenden Oberfläche in Kombination mit einer geeigneten Auswahl einer durch das Heizelement eingestellten Temperatur jeweilige durch den Spaltvorgang erzeugte Spaltprodukte beeinflusst werden, sodass durch den Spaltvorgang bspw. Stickoxide oder Cyanwasserstoffe entstehen.

Die Oberfläche umfasst mindestens ein Material ausgewählt aus der folgenden Liste bestehend aus: Ruthenium und Rhodium.

Je nach Wahl des Materials bzw. der Materialien für die erfindungsgemäß vorgesehene Oberfläche wird mehr oder weniger thermische Energie zum thermischen Spalten benötigt. Entsprechend kann je nach Auswahl des Materials bzw. einer Materialkombination ein entsprechend geeignetes Heizelement gewählt werden, das vorzugsweise einen minimalen Energieverbrauch hat.

Das Material bzw. die Materialkombination der erfindungsgemäß vorgesehenen Oberfläche kann direkt oder geträgert auf Aluminuimoxid, Zirkoniumoxid, Siliziumoxid, Ceroxid oder Keramik vorgesehen sein.

Es kann weiterhin vorgesehen sein, dass der Sensor und das Heizelement zu einem integrierten Bauteil zusammengefasst sind und das Heizelement dazu konfiguriert ist, eine Oberfläche des Bauteils zu erwärmen.

Mittels eines integrierten Bauteils, das bspw. ein Pellistor mit einer Pellistorperle sein kann, kann eine kompakte und energetisch effiziente Messeinheit bereitgestellt werden. Dabei kann eine äußere Oberfläche der Pellistorperle aus einem katalytischen Material bestehen, das eine für einen thermischen Spaltvorgang benötigte Energiemenge reduziert.

Es kann weiterhin vorgesehen sein, dass das Heizelement oder eine Kombination aus Heizelement und Oberfläche dazu konfiguriert ist, in der Probe enthaltenes Dicyan zu Stickoxid oder zu Cyanwasserstoff zu spalten.

Durch eine gemeinsame Wirkung von Heizelement und katalytischer Oberfläche kann ein bei einem thermischen Spaltvorgang erzeugtes Spaltprodukt genau vorgegeben werden. Insbesondere kann die katalytische Oberfläche bzw. ein durch das Heizelement bereitgestellter Energieeintrag derart bemessen werden, dass Stickoxide oder Cyanwasserstoffe entstehen.

Es kann weiterhin vorgesehen sein, dass die Messkammer eine für Dicyan durchlässige und für Cyanwasserstoff undurchlässige Filtereinheit umfasst.

Um einen Einfluss von Cyanwasserstoff auf eine Detektion von Dicyan zu minimieren, kann das vorgestellte Gasmessgerät einen Filter umfassen, der einen Eintritt von Cyanwasserstoff in die Kammer des Gasmessgeräts verhindert. Alternativ kann die Filtereinheit eine Membran, wie bspw. eine PTFE-Membran sein, die durchlässig für Cyanwasserstoff und Dicyan ist und die dazu konfiguriert ist, Strömungseinflüsse auf eine Detektion mittels des erfindungsgemäß vorgesehenen Sensors zu minimieren.

Es kann weiterhin vorgesehen sein, dass das Gasmessgerät eine Pumpe zum Einbringen der Probe in die Messkammer umfasst.

Das vorgestellte Gasmessgerät kann als passives Gasmessgerät auf dem Prinzip der Diffusion beruhen oder eine Pumpe umfassen, mittels derer eine Probe aktiv aus einer Umgebung entnommen und in die Kammer des Gasmessgeräts eingebracht werden kann.

Es kann weiterhin vorgesehen sein, dass das Gasmessgerät einen Nebensensor umfasst, wobei der Sensor dazu konfiguriert ist, Stickoxide zu detektieren und der Nebensensor dazu konfiguriert ist, Cyanwasserstoff zu detektieren.

Mittels zweier Sensoren, also einem Sensor zum Erfassen von Stickoxiden und einem Nebensensor zum Erfassen von Cyanwasserstoff können Konzentrationen beider Gase, also von Dicyan und Cyanwasserstoff ermittelt werden.

In einem zweiten Aspekt betrifft die vorgestellte Erfindung ein Verfahren zum Messen von Dicyan in Anwesenheit von Cyanwasserstoff, wobei das Verfahren einen Bereitstellungsschritt zum Bereitstellen einer möglichen Ausgestaltung des vorgestellten Gasmessgeräts, einen Zuführungsschritt zum Zuführen der Probe in die Messkammer des Gasmessgeräts, einen Spaltungsschritt zum thermischen Spalten von in der Probe befindlichem Dicyan mittels des Heizelements des Gasmessgeräts und einen Detektionsschritt zum Detektieren von durch den Spaltungsschritt erzeugten Spaltprodukten des Dicyans mittels des Sensors des Gasmessgeräts umfasst.

Das vorgestellte Verfahren dient insbesondere zum Betrieb des vorgestellten Gasmessgeräts.

Es kann vorgesehen sein, dass das Verfahren weiterhin einen Detektionsschritt zum Detektieren von Cyanwasserstoff umfasst. Das Detektieren von Cyanwasserstoff kann zusätzlich zum Detektieren von Spaltprodukten verwendet werden, um neben Informationen zu einer Konzentration von Dicyan Informationen über eine Konzentration von Cyanwasserstoff in der Probe zu ermitteln. Alternativ kann das Detektieren von Cyanwasserstoff durchgeführt werden, um Spaltprodukte an sich zu detektieren.

Es kann weiterhin vorgesehen sein, dass das Detektieren von Cyanwasserstoff mittels des Sensors in einem ersten Erfassungsschritt zeitlich vor dem thermischen Spalten durchgeführt wird und in einem zweiten Erfassungsschritt zeitlich nach dem thermischen Spalten durchgeführt wird.

### BEVORZUGTE AUSFÜHRUNGSBEISPIELE

Weitere, die Erfindung verbessernde Maßnahmen ergeben sich aus der nachfolgenden Beschreibung zu einigen Ausführungsbeispielen der Erfindung, welche in den Figuren dargestellt sind.

Es zeigen jeweils schematisch:
- Figur 1: eine schematische Darstellung einer möglichen Ausgestaltung des erfindungsgemäßen Gasmessgeräts,
- Figur 2: eine schematische Darstellung einer möglichen Ausgestaltung des erfindungsgemäß vorgesehenen Sensors,
- Figur 3: eine schematische Darstellung eines Ablaufs des erfindungsgemäßen Verfahrens.

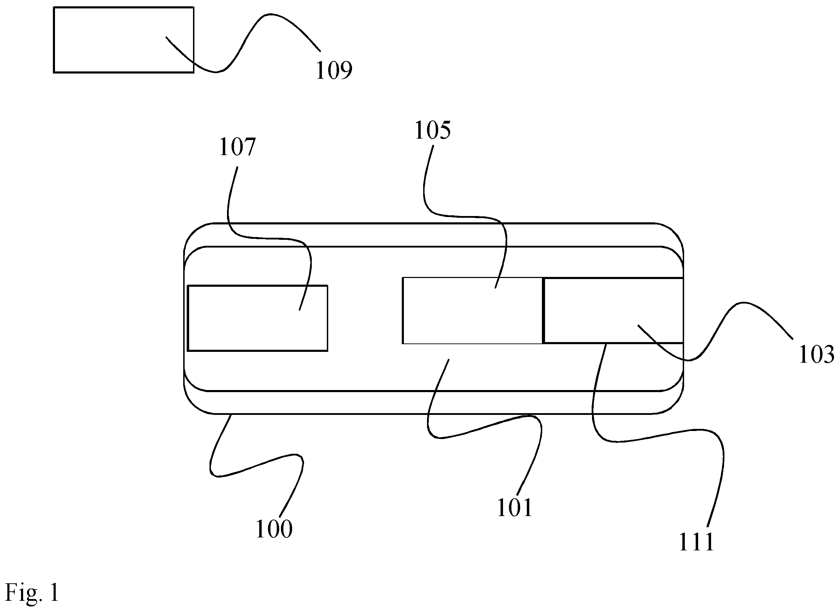

In Fig. 1 ist ein Gasmessgerät 100 dargestellt. Das Gasmessgerät 100 umfasst eine Messkammer 101, ein Heizelement 103 und einen elektrochemischen Sensor 105.

Zum Messen einer Konzentration von Dicyan in Anwesenheit von Cyanwasserstoff wird eine in der Messkammer 101 befindliche Probe mittels des Heizelements 103 erwärmt und, dadurch bedingt, thermisch gespalten. Die durch das thermische Spalten entstandenen Spaltprodukte werden mittels des Sensors 105 detektiert. Anhand von durch den Sensor 105 ermittelten Messwerten kann bspw. unter Verwendung einer optionalen Recheneinheit 107 auf eine Konzentration von Dicyan in der Probe geschlossen werden.

Alternativ können durch den Sensor 105 ermittelte Messwerte direkt zum Darstellen einer Konzentration von Dicyan in der Probe verwendet werden. Dazu kann der Sensor 105 bspw. mit einer Ausgabeeinheit 109, wie bspw. einer Anzeige und/oder einem Lautsprecher verbunden sein.

Es kann vorgesehen sein, dass die Recheneinheit 107 dazu konfiguriert ist, mittels der Ausgabeeinheit 109 eine Warnung auszugeben, wenn eine in einer jeweiligen Probe detektierte Konzentration an Dicyan oder Cyanwasserstoff über einem vorgegebenen Schwellenwert liegt.

Vorliegend ist das Gasmessgerät 100 ein mobiles bzw. tragbares Gasmessgerät mit einer Stromquelle, sodass das Gasmessgerät "im Feld" einsetzbar ist.

Um einen Energiebedarf für ein thermisches Spalten von Dicyanmolekülen in der Probe zu minimieren bzw. einen Spaltprozess in ausgewählte Spaltprodukte, wie bspw. Stickoxide oder Cyanwasserstoff zu kontrollieren bzw. zu steuern, ist in der Kammer 101, insbesondere an dem Heizelement 103 die katalytische Oberfläche 111 angeordnet.

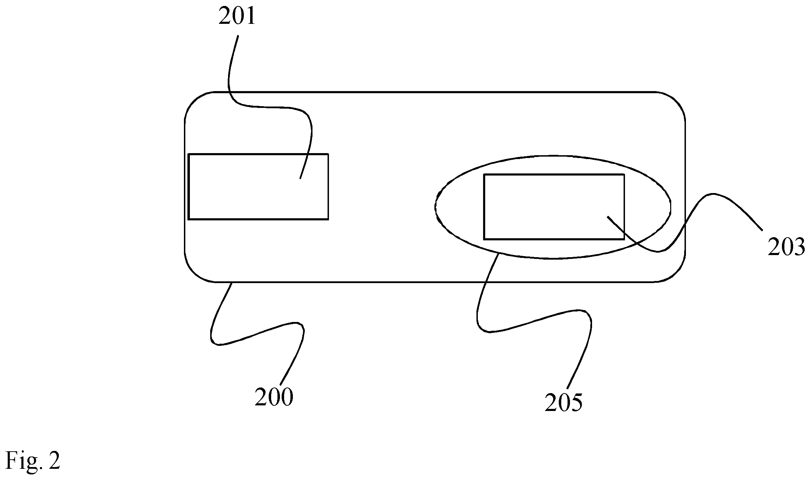

In Fig. 2 ist ein Sensor 200 dargestellt. Der Sensor 200 ist ein integriertes Bauteil und umfasst eine Messelektrode 201, ein Heizelement 203 und die katalytische Oberfläche 205 in Form einer Pellistorperle, die das Heizelement 203 umgibt bzw. als integraler Bestandteil des Heizelements 203 ausgestaltet ist. Entsprechend wird durch das Heizelement 203 erzeugte Wärmenergie auf die katalytische Oberfläche 205 übertragen.

Sobald ein Dicyanmolekül mit der katalytischen Oberfläche 205 in Kontakt kommt, wird das Dicyanmolekül aufgrund der in die katalytische Oberfläche 205 eingebrachten Wärmeenergie und aufgrund der katalytischen Eigenschaften der katalytischen Oberfläche 205 thermisch bspw. in Stickstoffdioxid und Kohlendioxid gespalten.

Der Sensor 200 ist spezifisch zur Detektion von Stickstoffdioxid konfiguriert und ermittelt entsprechend einen Messwert in Abhängigkeit einer gemessenen Konzentration an Stickstoffdioxid. Entsprechend ist der ermittelte Messwert proportional zu einer Konzentration an Dicyan und ermöglicht eine Beurteilung darüber, ob eine Dicyankonzentration in einer Umgebung über oder unter einem vorgegebenen Schwellenwert liegt.

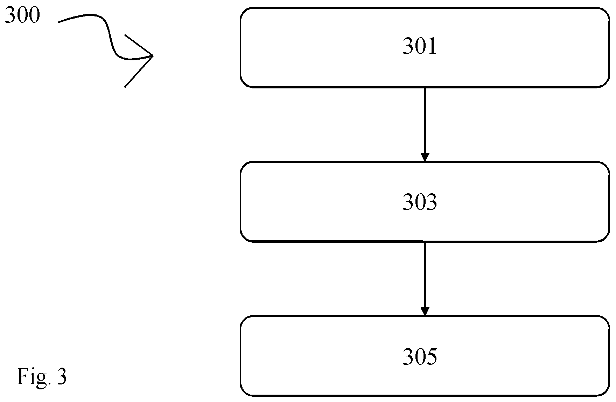

In Fig. 3 ist ein Verfahren 300 dargestellt. Das Verfahren 300 umfasst einen Bereitstellungsschritt 301 zum Bereitstellen einer möglichen Ausgestaltung des vorgestellten Gasmessgeräts, einen Zuführungsschritt 303 zum Zuführen der Probe in die Messkammer des Gasmessgeräts, einen Spaltungsschritt zum thermischen Spalten von in der Probe befindlichem Dicyan mittels des Heizelements des Gasmessgeräts und einen Detektionsschritt 305 zum Detektieren von durch den Spaltungsschritt erzeugten Spaltprodukten des Dicyans mittels des Sensors des Gasmessgeräts.

### BEZUGSZEICHENLISTE

- 100: Gasmessgerät
- 101: Messkammer
- 103: Heizelement
- 105: Sensor
- 107: Recheneinheit
- 109: Ausgabeeinheit
- 111: katalytische Oberfläche
- 200: Sensor
- 201: Messelektrode
- 203: Heizelement
- 205: katalytische Oberfläche
- 300: Verfahren
- 301: Bereitstellungsschritt
- 303: Zuführungsschritt
- 305: Detektionsschritt

## Patentansprüche

1. Verwendung eines Gasmessgeräts (100) zum Messen von Dicyan in Anwesenheit von Cyanwasserstoff,
wobei das Gasmessgerät (100) umfasst:
- eine Messkammer (101),
- ein Heizelement (103, 203),
- einen elektrochemischen Sensor (105, 200),
wobei die Messkammer (101) eine Probe enthaltend Dicyan aufnimmt, wobei das Heizelement (103) das in der Probe enthaltene Dicyan thermisch in Spaltprodukte spaltet,
wobei der Sensor (105, 200) die durch das thermische Spalten erhaltenen Spaltprodukte des Dicyans detektiert, wobei das Gasmessgerät (100) eine Oberfläche (111, 205) aufweist, die als Katalysator bei einer thermischen Spaltung von Dicyan wirkt und wobei die Oberfläche (111, 205) mindestens ein Material der folgenden Liste an Materialien umfasst: Ruthenium und Rhodium.

2. Verwendung (100) nach Anspruch 1, wobei
das Heizelement (103, 203) in der Probe enthaltenes Dicyan zumindest teilweise in Stickoxide spaltet, und
der Sensor (105, 200) die Stickoxide detektiert.

3. Verwendung nach Anspruch 1 oder 2, wobei
das Gasmessgerät (100) eine Recheneinheit (107) umfasst, die dazu konfiguriert ist, anhand von durch den Sensor (105, 200) ermittelten Messwerten eine Konzentration von in der Probe enthaltenem Cyanwasserstoff zu berechnen, oder
das Gasmessgerät (100) eine Recheneinheit (107) umfasst und der Sensor (105, 200) dazu konfiguriert ist, Cyanwasserstoff zu detektieren, wobei die Recheneinheit (107) dazu konfiguriert ist, anhand von während eines ersten Zeitbereichs vor einer thermischen Spaltung durch das Heizelement (103 203) mittels des Sensors (105, 200) ermittelten Messwerten eine Konzentration von in der Probe enthaltenem Cyanwasserstoff und anhand von während eines zweiten Zeitbereichs nach einer thermischen Spaltung durch das Heizelement (103, 203) mittels des Sensors (105, 200) ermittelten Messwerten eine Konzentration von in der Probe enthaltenem Dicyan zu berechnen.

4. Verwendung nach einem der voranstehenden Ansprüche, wobei der Sensor (105, 200) und das Heizelement (103, 203) zu einem integrierten Bauteil zusammengefasst sind und das Heizelement (103, 203) eine Oberfläche des Bauteils erwärmt.

5. Verwendung nach einem der voranstehenden Ansprüche, wobei das Heizelement (103, 203) oder eine Kombination aus Heizelement (103, 203) und Oberfläche (111, 205), in der Probe enthaltenes Dicyan zu Stickoxid oder zu Cyanwasserstoff spaltet.

6. Verwendung nach einem der voranstehenden Ansprüche, wobei die Messkammer (101) eine für Dicyan durchlässige und für Cyanwasserstoff undurchlässige Filtereinheit umfasst.

7. Verwendung nach einem der voranstehenden Ansprüche, wobei das Gasmessgerät (100) eine Pumpe zum Einbringen einer Probe in die Messkammer (101) umfasst.

8. Verwendung nach einem der voranstehenden Ansprüche, wobei der Sensor (200) oder das Heizelement (103, 203) eine Pellistorperle umfasst.

9. Verwendung nach einem der voranstehenden Ansprüche, wobei das Gasmessgerät (100) einen Nebensensor umfasst, wobei der Sensor (105, 200) Stickoxide und der Nebensensor Cyanwasserstoff detektiert.

10. Verfahren (300) zum Messen von Dicyan in Anwesenheit von Cyanwasserstoff, wobei das Verfahren umfasst:
- Bereitstellen (301) eines Gasmessgeräts (100), wobei das Gasmessgerät (100) eine Messkammer (101), ein Heizelement (103, 203), einen elektrochemischen Sensor (105, 200) und eine Oberfläche (111, 205) aufweist, die als Katalysator bei einer thermischen Spaltung von Dicyan wirkt und mindestens ein Material der folgenden Liste an Materialien umfasst: Ruthenium und Rhodium,
- Zuführen (303) einer Probe umfassend Dicyan und Cyanwasserstoff in die Messkammer (101) des Gasmessgeräts (100),
- thermisches Spalten (303) von in der Probe befindlichem Dicyan mittels des Heizelements (103, 203) des Gasmessgeräts (100),
- Detektieren (305) von durch das thermische Spalten erzeugten Spaltprodukten des Dicyans mittels des Sensors (105, 200) des Gasmessgeräts (100).

11. Verfahren (300) nach Anspruch 10, wobei
das Verfahren (300) weiterhin umfasst:
- Detektieren von Cyanwasserstoff.

12. Verfahren (300) nach Anspruch 11, wobei
das Detektieren von Cyanwasserstoff mittels des Sensors (105, 200) in einem ersten Erfassungsschritt zeitlich vor dem thermischen Spalten (303) durchgeführt wird und in einem zweiten Erfassungsschritt zeitlich nach dem thermischen Spalten (303) durchgeführt wird.

13. Verfahren nach einem der Ansprüche 10 bis 12, wobei der Sensor (200) oder das Heizelement (103, 203) eine Pellistorperle umfasst.

14. Verfahren nach einem der Ansprüche 11 bis 13, wobei das Gasmessgerät (100) einen Nebensensor umfasst, wobei der Sensor (105, 200) Stickoxide und der Nebensensor Cyanwasserstoff detektiert.

15. Verfahren nach einem der Ansprüche 11 bis 14, wobei das Gasmessgerät (100) eine Recheneinheit (107) umfasst, die anhand von durch den Sensor (105, 200) ermittelten Messwerten eine Konzentration von in der Probe enthaltenem Cyanwasserstoff berechnet, oder
das Gasmessgerät (100) eine Recheneinheit (107) umfasst und der Sensor (105, 200) Cyanwasserstoff detektiert,
wobei die Recheneinheit (107) anhand von während eines ersten Zeitbereichs vor einer thermischen Spaltung durch das Heizelement (103 203) mittels des Sensors (105, 200) ermittelten Messwerten eine Konzentration von in der Probe enthaltenem Cyanwasserstoff und anhand von während eines zweiten Zeitbereichs nach einer thermischen Spaltung durch das Heizelement (103, 203) mittels des Sensors (105, 200) ermittelten Messwerten eine Konzentration von in der Probe enthaltenem Dicyan berechnet.

## Claims

1. Use of a gas measuring device (100) for measuring cyanogen in the presence of hydrogen cyanide,
wherein the gas measuring device (100) comprises:
- a measuring chamber (101),
- a heating element (103, 203),
- an electrochemical sensor (105, 200),
wherein the measuring chamber (101) receives a sample containing cyanogen,
wherein the heating element (103) thermally decomposes the cyanogen contained in the sample into decomposition products,
wherein the sensor (105, 200) detects the decomposition products of the cyanogen that are obtained by the thermal decomposition, wherein the gas measuring device (100) has a surface (111, 205) that acts as a catalyst in a process of thermal decomposition of cyanogen and wherein the surface (111, 205) comprises at least one material from the following list of materials: ruthenium and rhodium.

2. Use (100) according to claim 1, wherein
the heating element (103, 203) decomposes cyanogen contained in the sample at least partially into nitrogen oxides, and
the sensor (105, 200) detects the nitrogen oxides.

3. Use according to claim 1 or 2, wherein
the gas measuring device (100) comprises a computing unit (107) configured to calculate a concentration of hydrogen cyanide contained in the sample on the basis of measured values determined by the sensor (105, 200), or
the gas measuring device (100) comprises a computing unit (107) and the sensor (105, 200) is configured to detect hydrogen cyanide,
wherein the computing unit (107) is configured to calculate a concentration of hydrogen cyanide contained in the sample on the basis of measured values determined by means of the sensor (105, 200) during a first time period before a process of thermal decomposition by the heating element (103 203) and to calculate a concentration of cyanogen contained in the sample on the basis of measured values determined by means of the sensor (105, 200) during a second time period after a process of thermal decomposition by the heating element (103, 203).

4. Use according to any of the preceding claims, wherein
the sensor (105, 200) and the heating element (103, 203) are combined to form an integrated component and the heating element (103, 203) heats a surface of the component.

5. Use according to any of the preceding claims, wherein
the heating element (103, 203) or a combination of the heating element (103, 203) and the surface (111, 205) decomposes cyanogen contained in the sample into nitrogen oxide or into hydrogen cyanide.

6. Use according to any of the preceding claims, wherein
the measuring chamber (101) comprises a filter unit that is permeable to cyanogen and impermeable to hydrogen cyanide.

7. Use according to any of the preceding claims, wherein
the gas measuring device (100) comprises a pump for introducing a sample into the measuring chamber (101).

8. Use according to any of the preceding claims, wherein
the sensor (200) or the heating element (103, 203) comprises a pellistor bead.

9. Use according to any of the preceding claims, wherein
the gas measuring device (100) comprises an auxiliary sensor, wherein the sensor (105, 200) detects nitrogen oxides and the auxiliary sensor detects hydrogen cyanide.

10. Method (300) for measuring cyanogen in the presence of hydrogen cyanide, wherein the method comprises:
- providing (301) a gas measuring device (100), wherein the gas measuring device (100) has a measuring chamber (101), a heating element (103, 203), an electrochemical sensor (105, 200) and a surface (111, 205) that acts as a catalyst in a process of thermal decomposition of cyanogen and comprises at least one material from the following list of materials: ruthenium and rhodium,
- feeding (303) a sample comprising cyanogen and hydrogen cyanide into the measuring chamber (101) of the gas measuring device (100),
- thermally decomposing (303) cyanogen present in the sample by means of the heating element (103, 203) of the gas measuring device (100),
- detecting (305), by means of the sensor (105, 200) of the gas measuring device (100), decomposition products of the cyanogen that are produced by the thermal decomposition.

11. Method (300) according to claim 10, wherein
the method (300) furthermore comprises:
- detecting hydrogen cyanide.

12. Method (300) according to claim 11, wherein
the step of detecting hydrogen cyanide by means of the sensor (105, 200) is carried out in a first sensing step chronologically before the thermal decomposition (303) and in a second sensing step chronologically after the thermal decomposition (303).

13. Method according to any of claims 10 to 12, wherein
the sensor (200) or the heating element (103, 203) comprises a pellistor bead.

14. Method according to any of claims 11 to 13, wherein
the gas measuring device (100) comprises an auxiliary sensor, wherein the sensor (105, 200) detects nitrogen oxides and the auxiliary sensor detects hydrogen cyanide.

15. Method according to any of claims 11 to 14, wherein
the gas measuring device (100) comprises a computing unit (107) that calculates a concentration of hydrogen cyanide contained in the sample on the basis of measured values determined by the sensor (105, 200), or
the gas measuring device (100) comprises a computing unit (107) and the sensor (105, 200) detects hydrogen cyanide,
wherein the computing unit (107) calculates a concentration of hydrogen cyanide contained in the sample on the basis of measured values determined by means of the sensor (105, 200) during a first time period before a process of thermal decomposition by the heating element (103 203) and calculates a concentration of cyanogen contained in the sample on the basis of measured values determined by means of the sensor (105, 200) during a second time period after a process of thermal decomposition by the heating element (103, 203).

## Revendications

1. Utilisation d'un appareil compteur à gaz (100) pour la mesure du cyanogène en présence de cyanure d'hydrogène,
dans laquelle l'appareil compteur à gaz (100) comprend :
- une chambre de mesure (101),
- un élément chauffant (103, 203),
- un capteur électrochimique (105, 200),
dans laquelle la chambre de mesure (101) reçoit un échantillon contenant du cyanogène,
dans laquelle l'élément chauffant (103) sépare thermiquement le cyanogène contenu dans l'échantillon en produits de séparation,
dans laquelle le capteur (105, 200) détecte les produits de séparation du cyanogène obtenus par la séparation thermique, dans laquelle l'appareil compteur à gaz (100) présente une surface (111, 205) qui agit comme un catalyseur lors d'une séparation thermique du cyanogène et dans laquelle la surface (111, 205) comprend au moins un matériau de la liste suivante de matériaux : ruthénium et rhodium.

2. Utilisation (100) selon la revendication 1, dans laquelle
l'élément chauffant (103, 203) sépare au moins partiellement le cyanogène contenu dans l'échantillon en oxydes d'azote, et
le capteur (105, 200) détecte les oxydes d'azote.

3. Utilisation selon la revendication 1 ou 2, dans laquelle
l'appareil compteur à gaz (100) comprend une unité de calcul (107) qui est configurée pour calculer une concentration de cyanure d'hydrogène contenu dans l'échantillon à l'aide de valeurs de mesure déterminées par le capteur (105, 200), ou
l'appareil compteur à gaz (100) comprend une unité de calcul (107) et le capteur (105, 200) est configuré pour détecter le cyanure d'hydrogène,
dans laquelle l'unité de calcul (107) est configurée pour calculer une concentration de cyanure d'hydrogène contenu dans l'échantillon à l'aide de valeurs de mesure déterminées au moyen du capteur (105, 200) pendant une première plage temporelle avant une séparation thermique par l'élément chauffant (103, 203), et une concentration de cyanogène contenu dans l'échantillon à l'aide de valeurs de mesure déterminées au moyen du capteur (105, 200) pendant une seconde plage temporelle après une séparation thermique par l'élément chauffant (103, 203).

4. Utilisation selon l'une des revendications précédentes, dans laquelle
le capteur (105, 200) et l'élément chauffant (103, 203) sont regroupés en un composant intégré et l'élément chauffant (103, 203) chauffe une surface du composant.

5. Utilisation selon l'une des revendications précédentes, dans laquelle
l'élément chauffant (103, 203) ou une combinaison de l'élément chauffant (103, 203) et de la surface (111, 205) sépare le cyanogène contenu dans l'échantillon en oxyde d'azote ou en cyanure d'hydrogène.

6. Utilisation selon l'une des revendications précédentes, dans laquelle
la chambre de mesure (101) comprend une unité filtrante perméable au cyanogène et imperméable au cyanure d'hydrogène.

7. Utilisation selon l'une des revendications précédentes, dans laquelle
l'appareil compteur à gaz (100) comprend une pompe pour l'introduction d'un échantillon dans la chambre de mesure (101).

8. Utilisation selon l'une des revendications précédentes, dans laquelle
le capteur (200) ou l'élément chauffant (103, 203) comprend une perle de pellistor.

9. Utilisation selon l'une des revendications précédentes, dans laquelle
l'appareil compteur à gaz (100) comprend un capteur auxiliaire, dans laquelle le capteur (105, 200) détecte les oxydes d'azote et le capteur auxiliaire détecte le cyanure d'hydrogène.

10. Procédé (300) pour la mesure du cyanogène en présence de cyanure d'hydrogène, dans lequel le procédé comprend :
- la fourniture (301) d'un appareil compteur à gaz (100), dans lequel l'appareil compteur à gaz (100) présente une chambre de mesure (101), un élément chauffant (103, 203), un capteur électrochimique (105, 200) et une surface (111, 205) qui agit comme un catalyseur lors d'une séparation thermique du cyanogène et comprend au moins un matériau de la liste de matériaux suivante : ruthénium et rhodium,
- l'amenée (303) d'un échantillon comprenant du cyanogène et du cyanure d'hydrogène dans la chambre de mesure (101) de l'appareil compteur à gaz (100),
- la séparation thermique (303) du cyanogène présent dans l'échantillon au moyen de l'élément chauffant (103, 203) de l'appareil compteur à gaz (100),
- la détection (305), au moyen du capteur (105, 200) de l'appareil compteur à gaz (100), des produits de séparation du cyanogène générés par la séparation thermique.

11. Procédé (300) selon la revendication 10, dans lequel
le procédé (300) comprend en outre :
- la détection du cyanure d'hydrogène.

12. Procédé (300) selon la revendication 11, dans lequel
la détection du cyanure d'hydrogène au moyen du capteur (105, 200) est effectuée dans une première étape de détection périodiquement avant la séparation thermique (303) et est effectuée dans une seconde étape de détection périodiquement après la séparation thermique (303).

13. Procédé selon l'une des revendications 10 à 12, dans lequel
le capteur (200) ou l'élément chauffant (103, 203) comprend une perle de pellistor.

14. Procédé selon l'une des revendications 11 à 13, dans lequel
l'appareil compteur à gaz (100) comprend un capteur auxiliaire, dans lequel le capteur (105, 200) détecte les oxydes d'azote et le capteur auxiliaire détecte le cyanure d'hydrogène.

15. Procédé selon l'une des revendications 11 à 14, dans lequel
l'appareil compteur à gaz (100) comprend une unité de calcul (107) qui calcule une concentration de cyanure d'hydrogène contenu dans l'échantillon à l'aide de valeurs de mesure déterminées par le capteur (105, 200), ou
l'appareil compteur à gaz (100) comprend une unité de calcul (107) et le capteur (105, 200) détecte du cyanure d'hydrogène,
dans lequel l'unité de calcul (107) calcule une concentration de cyanure d'hydrogène contenu dans l'échantillon à l'aide de valeurs de mesure déterminées au moyen du capteur (105, 200) pendant une première plage temporelle avant une séparation thermique par l'élément chauffant (103, 203), et une concentration de cyanogène contenu dans l'échantillon à l'aide de valeurs de mesure déterminées au moyen du capteur (105, 200) pendant une seconde plage temporelle après une séparation thermique par l'élément chauffant (103, 203).
